# FASCICULE DE BREVET EUROPEEN

(11) **EP 2 731 584 B1**
(45) Date de publication et mention de la délivrance du brevet: **11.01.2017**
(21) Numéro de dépôt: 12743514.7
(22) Date de dépôt: 12.07.2012
(51) Int. Cl.: A61K 8/60, A61K 31/7016, A61L 15/16, A61Q 19/00, A61P 17/02, A61L 15/22, A61L 15/44, A61K 8/34, A61K 8/36, A61K 8/37, A61K 8/891, A61K 9/00, A61K 9/70, A61L 15/28, A61K 31/415, A61K 31/513

(54) **UTILISATION DE COMPOSES OLIGOSACCHARIDIQUES POUR LA PREVENTION ET LE TRAITEMENT DES CICATRICES PATHOLOGIQUES**
VERWENDUNG VON OLIGOSACCHARIDEN ZUR PRÄVENTION UND BEHANDLUNG VON PATHOLOGISCHEN NARBEN
USE OF OLIGOSACCHARIDE COMPOUNDS FOR THE PREVENTION AND TREATMENT OF PATHOLOGICAL SCARS

(30) Priorité: 13.07.2011 FR 1156436
(43) Date de publication de la demande: 21.05.2014
(73) Titulaire: URGO RECHERCHE INNOVATION ET DEVELOPPEMENT, 21300 Chenôve (FR)
(72) Inventeur: BOUSCHBACHER, Marielle, F-21220 Chambolle-Musigny (FR); LAURENSOU, Christelle, F-21000 Dijon (FR)
(74) Mandataire: Cabinet Plasseraud
(86) Numéro de dépôt international: PCT/FR2012/051668
(87) Numéro de publication internationale: WO 2013/007960

(56) Documents cités:
- WO-A1-02/089818
- FR-A1- 2 824 474
- FR-A1- 2 916 355
- FR-A1- 2 953 522
- BIROL CIVELEK ET AL: "The effect of sucralfate, an agent for gastroprotection on the healing of split thickness skin graft donor sites", EUROPEAN JOURNAL OF PLASTIC SURGERY, SPRINGER, BERLIN, DE, vol. 30, no. 1, 28 avril 2007 (2007-04-28) , pages 25-28, XP019513152, ISSN: 1435-0130, DOI: 10.1007/S00238-007-0140-Z

## Description

La présente invention a pour objet un composé choisi parmi les oligosaccharides polysulfatés synthétiques ayant 1 à 4 unités oses, leurs sels, ou leurs complexes, pour son utilisation pour traiter des plaies générant des cicatrices pathologiques.

La cicatrisation d'une plaie est un phénomène biologique naturel, les tissus humains et animaux étant capables de réparer des lésions localisées par des processus de réparation et de régénération qui leur sont propres.

La rapidité et la qualité de la cicatrisation d'une plaie dépendent de l'état général de l'organisme atteint, de l'étiologie de la plaie, de l'état et de la localisation de la plaie, et de la survenue ou non d'une infection, ainsi que des facteurs génétiques prédisposant ou non à des troubles de la cicatrisation.

La cicatrisation naturelle d'une plaie se déroule principalement selon trois phases successives, chacune de ces phases étant caractérisée par des activités cellulaires spécifiques qui font progresser le processus de réparation selon des séquences chronologiques précises : la phase inflammatoire, la phase de granulation (ou phase proliférative), et la phase de formation de la cicatrice.

La première phase, la phase inflammatoire, débute dès la rupture des vaisseaux sanguins qui déclenche la formation d'un caillot (coagulation du sang) principalement composé de fibrine et de fibronectine, et qui va constituer une matrice provisoire. Cette matrice comble en partie la lésion et va permettre la migration au sein de la zone lésée des cellules inflammatoires recrutées pour assurer la détersion de la plaie. Les plaquettes présentes vont également libérer des facteurs (par exemple cytokine, facteurs de croissance) permettant le recrutement des cellules de la cicatrisation comme les cellules inflammatoires (les polynucléaires neutrophiles et les macrophages), les fibroblastes et les cellules endothéliales.

La seconde phase correspond au développement du tissu de granulation. On observe d'abord une colonisation de la blessure par prolifération des fibroblastes. Puis, la migration des cellules endothéliales à partir des vaisseaux sains va permettre la néovascularisation, ou angiogénèse, du tissu lésé. Dans le tissu de granulation, les fibroblastes sont activés et vont se différencier en myofibroblastes présentant des propriétés contractiles importantes, générées par les microfilaments d'actine, permettant la contraction de la plaie. Ces microfilaments sont exprimés par une protéine : l'actine α-musculaire lisse. Ces myofibroblastes jouent donc un rôle majeur dans la formation et la maturation du tissu de granulation qui va conduire à la cicatrisation de la lésion. Il y a ensuite migration des kératinocytes et reconstruction de l'épiderme.

La troisième phase du processus de réparation, la formation de la cicatrice ou maturation, s'accompagne d'un remodelage du tissu de granulation. Une partie de la matrice extracellulaire est digérée par des protéases (essentiellement des métallo-protéases matricielles (MMP) et des élastases), et on observe une réorganisation progressive de la matrice extracellulaire. Progressivement, le collagène de type III, majoritaire dans le tissu de granulation, est remplacé par le collagène de type I, principal composant matriciel du derme. A la fin de la phase de maturation, les fibroblastes, myofibroblastes et cellules vasculaires voient leur prolifération et/ou leur activité réduites. Puis les cellules excédentaires meurent par apoptose. Parallèlement au remodelage de la matrice extracellulaire et à l'apoptose des cellules excédentaires, l'état inflammatoire diminue progressivement. Cette phase est la plus longue : au bout d'un an environ, la cicatrice se remodèle, elle n'est plus rouge, ni rigide, et ne provoque plus douleur et elle s'aplanie.

Cependant, dans certains cas, la cicatrisation ne se passe pas aussi bien, et des cicatrices pathologiques peuvent se former. On parle alors de troubles de la cicatrisation. Ces derniers sont classiquement définis comme des dérèglements de la cicatrisation et regroupent plusieurs phénomènes :
- les ulcères chroniques, qui sont des plaies dont la cicatrisation est très prolongée dans le temps, ou qui ne cicatrisent pas du tout. Dans certains cas, les ulcères veineux par exemple, les plaies peuvent rester atones pendant plusieurs mois sans que le tissu de granulation ne se forme ni que la cicatrisation ne démarre (« Managing chronic venous leg ulcers: time for a new approach? » Brown A. J Wound Care. 2010 Feb;19(2):70-4. Review) ;
- les cicatrices atrophiques, présentant une perte de substance, résultent notamment de traumatismes externes ou de pathologies cutanées telles que l'acné sévère ou la varicelle. Ces cicatrices présentent des rétractions plus ou moins profondes de la surface de la peau, lui donnant ainsi un aspect grêlé (M. Chivot, H. Pawin, C. Beylot, O. Chosidow, B. Dreno, M. Faure, F. Poli, J. Revuz - Cicatrices d'acné : épidémiologie, physiopathologie, clinique, traitement - Ann. Dermatol. Venereol. 2006;133: 813-24) ;
- les cicatrices hypertrophiques dans lesquelles le tissu de granulation hyperprolifère de façon anormale (« Cellular and molecular pathology of HTS: basis for treatment. » Armour A, Scott PG, Tredget EE. Wound Repair Regen. 2007 Sep-Oct;15 Suppl 1:S6-17. Review. Erratum in: Wound Repair Regen. 2008 Jul-Aug;16(4):582) et qui peuvent notamment survenir lorsque la plaie atteint le derme réticulaire (également appelé le derme profond), riche en collagène ;
- les cicatrices rétractiles sont, quant à elles, dues à un rétrécissement de la zone cicatricielle provoquant une traction sur les tissus voisins. Elles sont le plus souvent consécutives à des brûlures dans certaines zones anatomiques spécifiques.

Les troubles de la cicatrisation regroupent donc des pathologies très différentes du processus normal de cicatrisation.

La présente invention s'intéresse plus particulièrement aux plaies engendrant les cicatrices hypertrophiques et les cicatrices rétractiles, voire les cicatrices atrophiques (cicatrices d'acné sévère).

Des méthodes de traitement des cicatrices pathologiques, une fois celles-ci formées, sont largement décrites dans la littérature. Il peut, par exemple, s'agir de "pressothérapie", réalisée avec des vêtements compressifs élastiques, ou de "corticothérapie" par injection à l'intérieur de la cicatrice de produits à base de cortisone. Pour les cicatrices atrophiques, des traitements par laser ou peeling sont souvent utilisés. Cependant ces traitements sont parfois invasifs, et nécessitent le plus souvent d'être suivis pendant plusieurs mois, voire des années, ce qui est contraignant pour le patient.

Le document « The effect of sucralfate, an agent for gastroprotection on the healing of split thickness graft donor sites" de Birol Civelek publié dans European Journal of Plastic Surgery (2007), vol. 30, pages 25-28, décrit le traitement par le sucralfate de plaies issues d'un prélèvement de peau en vue d'une greffe. Cependant, les plaies étudiées dans ce document ne peuvent pas générer de cicatrices hypertrophiques. En effet, comme explicité par exemple dans le document « Greffes cutanées » de Revol et al. publié dans Techniques chirurgicales - Chirurgie plastique reconstructrice et esthétique, (2010), 45-070, un greffon de peau mince doit être prélevé de façon à conserver des enclaves épithéliales dans le site donneur et à prélever des kératinocytes de la couche basale dans le greffon. Ainsi, le dermatome électrique doit passer au niveau des crêtes ou papilles dermiques ce qui revient à créer une plaie dans le derme papillaire, c'est-à-dire la couche superficielle du derme. Ainsi une plaie issue d'un prélèvement de greffon de peau mince n'atteint pas le derme réticulaire, c'est-à-dire le derme profond, et n'engendrent donc pas une désorganisation des parties profondes du derme. Ainsi les prélèvements de peau mince ne peuvent pas générer des cicatrices hypertrophiques. En revanche, il est connu que des cicatrices hypertrophiques peuvent avoir lieu sur un site donneur, lorsque l'on réalise une greffe de peau totale, c'est-à-dire que l'on prélève une couche de peau comprenant l'épiderme, le derme et ses annexes pilosébacées, ce qui n'est pas le cas dans ce document de l'art antérieur.

Enfin, il convient de noter que, dans le document Birol Civelek et al., que les plaies soient ou non traitées avec le sucralfate, elles ne développent pas de cicatrices hypertrophiques par la suite, ce qui confirme bien que ces plaies ne sont pas de nature à générer des cicatrices hypertrophiques, indépendamment de leur traitement.

Il serait donc souhaitable de traiter en amont, de manière préventive, les plaies générant des cicatrices pathologiques, de manière à limiter l'hyperprolifération ou la fibrose anormale du tissu de granulation lors de la cicatrisation, et ainsi réduire le risque de formation de cicatrices pathologiques.

L'invention a pour objet, selon un premier aspect, le sel de potassium du sucrose octasulfate, pour son utilisation pour traiter des plaies générant des cicatrices pathologiques, de préférence choisies parmi les cicatrices hypertrophiques ou rétractiles, voire les cicatrices atrophiques.

L'invention a également pour objet, selon un deuxième aspect, le sel de potassium du sucrose octasulfate, pour son utilisation pour traiter des plaies chez un sujet présentant une prédisposition au développement de cicatrices hypertrophiques ou rétractiles, voire les cicatrices atrophiques.

Selon un autre mode de réalisation, l'invention consiste en un sel de potassium de sucrose octasulfate pour son utilisation pour traiter des plaies générant des cicatrices pathologiques choisies parmi les cicatrices d'acné sévère.

Ce traitement est notamment adapté pour traiter des cicatrices pathologiques de toutes formes et de toutes tailles, quelle que soit leur localisation. De plus, contrairement aux traitements usuels appliqués après formation des cicatrices, le traitement selon la présente invention est utilisé de manière préventive sur des plaies présentant un risque de formation de cicatrices pathologiques.

Dans un processus normal de cicatrisation des plaies, il est important de favoriser le phénomène de fermeture de la plaie afin d'éviter, par exemple, l'invasion de la plaie par des microorganismes ou des substances étrangères, et par conséquent l'infection de la plaie. A titre d'exemple, le document FR-A-2916355 enseigne d'accélérer la prolifération et/ou la différenciation des fibroblastes en utilisant un copolymère particulier pour favoriser la vitesse de cicatrisation. Il est ensuite souhaitable de favoriser la migration des kératinocytes pour reconstituer l'épiderme.

Cependant, dans le cas particulier des cicatrices hypertrophiques, le tissu de granulation formé par les fibroblastes hyperprolifère de façon anormale. Lorsque les fibroblastes se différencient en myofibroblastes, cet excès de tissu fibreux se contracte, induisant des excroissances de tissu plus dense dans le derme.

Il n'est donc pas souhaitable, pour ce type de plaies spécifiques générant des cicatrices pathologiques hypertrophiques ou rétractiles, de favoriser la prolifération et la différenciation des myofibroblastes.

Il est au contraire souhaitable, pour ce type de plaies engendrant des cicatrices pathologiques, de contrôler les myofibroblastes en vue de limiter le développement d'une fibrose dermique.

Ainsi, l'invention a pour objet, selon un autre aspect, le sel de potassium du sucrose octasulfate, pour son utilisation pour inhiber la différentiation des fibroblastes en myofibroblastes au cours de la cicatrisation de plaies générant des cicatrices pathologiques choisies parmi les cicatrices hypertrophiques ou rétractiles, voire les cicatrices atrophiques

De façon surprenante, les inventeurs ont découvert que l'application du composé selon l'invention sur des plaies susceptibles de générer des cicatrices pathologiques permettait de prévenir efficacement la formation de cicatrices hypertrophiques, rétractiles ou atrophiques.

Cette application est particulièrement préférée chez des sujets présentant une prédisposition au développement de type de cicatrices pathologiques.

Les inventeurs ont en effet montré, dans le cadre de la présente demande, que l'inhibition de la différentiation des fibroblastes en myofibroblastes permettait de contrôler et/ou limiter l'hyperprolifération anormale du tissu de granulation, et/ou limiter la contraction de la plaie lors de la cicatrisation de plaies générant des cicatrices pathologiques, améliorant ainsi la qualité des cicatrices formées.

La présente invention a également mis en évidence que l'utilisation du sel de potassium du sucrose octasulfate influait sur la différentiation des fibroblastes en myofibroblastes.

### Description des figures

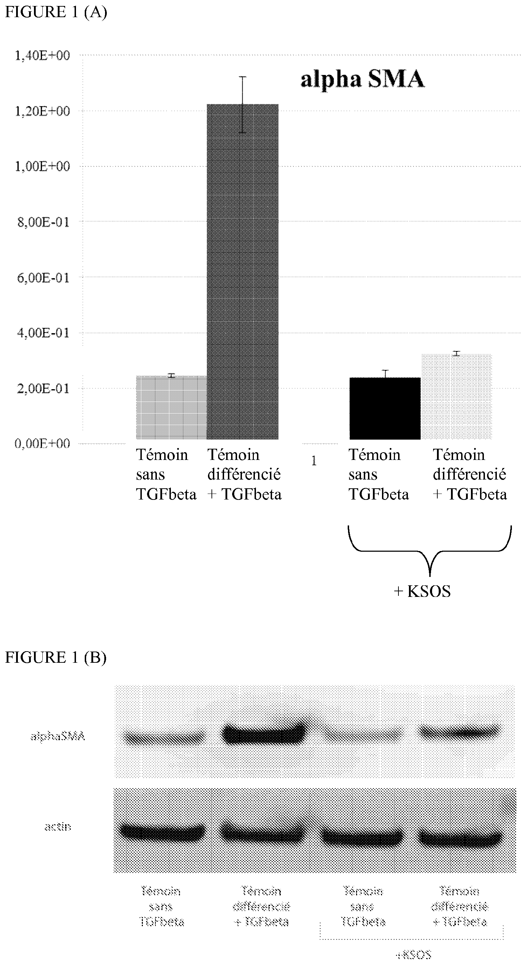

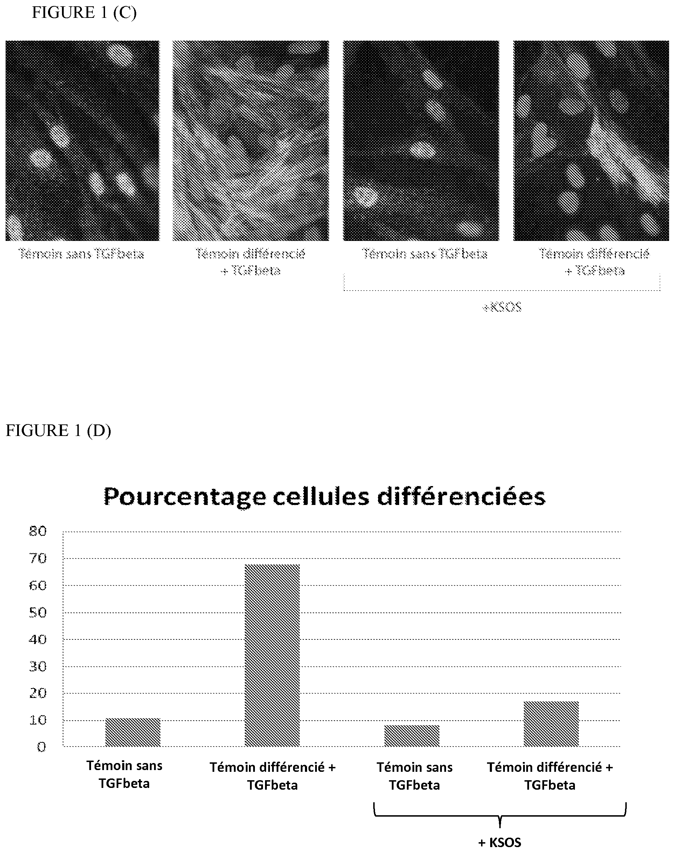

La figure 1 illustre l'effet du sel de potassium du sucrose octasulfate KSOS sur la différenciation des fibroblastes en myofibroblastes.

En particulier, la figure 1 (A) montre l'expression de l'ARN messager de l'α-SMA par RT-PCR quantitative dans 4 conditions (Témoin non différencié sans TGF-β, témoin différencié avec TGF-β, témoin non différencié sans TGF-β mais avec du KSOS, et témoin différencié avec TGF-β et du KSOS).

La figure 1 (B) montre l'expression protéique de l'α-SMA par la méthode de Western Blot.

La figure 1 (C) montre l'expression protéique de l'α-SMA visualisée par immunofluorescence.

La figure 1 (D) illustre les pourcentages de cellules différenciées exprimant l'α-SMA.

La figure 2 illustre, par des photographies des lattices de collagène à J1 et J7 dans les différentes conditions, l'effet inhibiteur de la contraction de lattices de collagène du pansement Urgotul® Start contenant le sel de potassium du sucrose octasulfate (KSOS) après 1 et 7 jours de culture.

### Cicatrices pathologiques

La présente invention s'intéresse aux plaies engendrant 3 types de cicatrices pathologiques: les cicatrices hypertrophiques, les cicatrices rétractiles et les cicatrices atrophiques.

Les cicatrices hypertrophiques ou rétractiles ont pour origine commune une phase hyperplasique initiale de forte intensité et/ou de longue durée, phase induisant un excès de tissu fibreux dense dans le derme non remanié. Ces cicatrices pathologiques sont boursouflées et volumineuses, rouges, dures et entrainent des démangeaisons. Elles sont caractérisées par un dépôt accru de collagène dermique, issu de la couche profonde du derme endommagée, de protéoglycanes, de fibronectine et eau tissulaire sous l'épithélium.

Les cicatrices hypertrophiques ou rétractiles se forment souvent après brûlure, mais peuvent aussi se développer après une blessure profonde (incision chirurgicale, par exemple). Elles restent confinées à l'intérieur des limites de la plaie originelle. Au niveau microscopique, elles présentent des fibres de collagène fines et des myofibroblastes exprimant l'actine α-musculaire lisse en grande quantité. Les rétractions observées dans les cicatrices hypertrophiques ou rétractiles sont provoquées par l'activité contractile des myofibroblastes. Ainsi, les myofibroblastes sont à l'origine de la désorganisation des faisceaux de collagène dans la couche profonde du derme. En effet, les faisceaux de collagène sont normalement plats mais, sous l'effet de la contraction des myofibroblastes, ils prennent une forme enroulée ce qui mène à la formation de nodules de collagène sous l'épithélium.

Les cicatrices hypertrophiques, lorsqu'elles sont situées au niveau d'une articulation (pli de flexion) ou d'une ligne de tension, peuvent, comme les cicatrices rétractiles, générer des rétractions invalidantes. La vascularisation y est plus abondante que dans la peau normale, et les vaisseaux sont plus dilatés. Après une phase active, qui peut durer un à deux ans, les cicatrices hypertrophiques peuvent régresser progressivement mais elles ne disparaissent totalement que rarement.

Les cicatrices rétractiles sont des cicatrices non fonctionnelles en ce sens qu'elles limitent la fonctionnalité de la zone sur laquelle elles se trouvent. Elles génèrent une perte de mobilité de la zone cicatricielle et des zones adjacentes, ce qui peut complètement limiter les mouvements (ex coude et mobilité du bras).

Les cicatrices atrophiques sont situées sous le niveau de la peau environnante. Elles forment de petits creux et apparaissent lorsque trop peu de nouvelles fibres de tissus conjonctifs sont produites durant le processus de cicatrisation. Les cicatrices d'acné sévère ou de varicelle sont des exemples typiques de cicatrices atrophiques. Au sens de la présente invention, par cicatrices d'acné sévère on entend des cicatrices d'acné qui sont générées par des lésions qui atteignent les couches du derme et pas seulement l'épiderme. Les cicatrices d'acné sévère sont le plus souvent le résultat de formes d'acné telles que l'acné nodulaire, l'acné fulminans, ou l'acné conglobata qui provoquent des lésions inflammatoires, telles que des nodules furonculoïdes indurés ayant un diamètre supérieur à 5mm (acné nodulaire) pouvant évoluer en fistules (acné conglobata). Les cicatrices d'acné sévère peuvent prendre des formes très variées. La première forme dite « en cratère » correspond à des cicatrices larges et rondes, avec une dépression simulant un cratère à fond plat. Les cicatrices d'acné en « pic à glace », petites et souvent plus profondes que les cicatrices « en cratère », donnent l'impression que la peau a été piquée par un instrument pointu. Elles sont les cicatrices les plus difficiles à traiter. Ces cicatrices sont le siège d'une fibrose qui fige la cicatrice et gêne les traitements usuels.

Il existe un certain nombre de facteurs favorisant l'apparition des cicatrices pathologiques, parmi les facteurs de risque de formation de cicatrices pathologiques, on peut citer :
- la carnation: les populations présentant une carnation foncée (d'origine maghrébine ou africaine) sont nettement plus sujettes aux cicatrices hypertrophiques que les populations caucasiennes. Les sujets présentant une carnation très claire ont par ailleurs également tendance à développer des cicatrices hypertrophiques ;
- l'âge : fréquentes chez les enfants, les cicatrices hypertrophiques sont rares chez les sujets âgés ;
- les hormones : certaines périodes de la vie, liées à de fortes poussées hormonales (puberté, grossesse), sont également plus propices au développement de cicatrices anormales, et aux cicatrices d'acné ;
- la localisation corporelle : certaines parties du corps sont plus sujettes à développer des cicatrices pathologiques, comme par exemple les zones de plis, les zones articulaires, les zones de tension (comme le coude, l'épaule, le thorax), le sternum, le cou, les oreilles ou le visage ;
- les pathologies infectieuses engendrant des cicatrices atrophiques (varicelle, acné sévère).

Les plaies générant des cicatrices pathologiques sont définies, au sens de la présente demande, comme les plaies atteignant le derme, de préférence des plaies atteignant le derme réticulaire (appelé également derme profond). Ainsi, les plaies générant des cicatrices pathologiques peuvent être définies comme des plaies ayant une profondeur supérieure à 0,30 mm, plus préférentiellement supérieure à 0,35 mm et plus préférentiellement encore supérieure à 0,40 mm.

Les plaies générant des cicatrices pathologiques peuvent également être définies au sens de la présente demande, comme étant des plaies localisées sur certaines parties du corps comme par exemple les zones de plis, les zones articulaires, les zones de tension (comme le coude, l'épaule, le thorax), le sternum, le cou, les oreilles ou le visage.

Les plaies générant des cicatrices pathologiques peuvent également être définies au sens de la présente demande, comme des plaies ouvertes apparaissant chez des sujets présentant une prédisposition au développement de cicatrices hypertrophiques, rétractiles ou cicatrices atrophiques, tels que par exemple les sujets présentant une carnation foncée ou très claire, les enfants, les adolescents en phase de puberté, les femmes enceintes.

Le sel de potassium du sucrose octasulfate selon l'invention présente donc un intérêt tout particulier pour le traitement des plaies chez des sujets présentant une prédisposition au développement de cicatrices hypertrophiques ou rétractiles ou atrophiques tels que les sujets présentant une carnation foncée ou très claire, les enfants, les adolescents en phase de puberté, les femmes enceintes.

### Sel de potassium du sucrose octasulfate

Le sel de potassium du sucrose octasulfate utilisé dans le cadre de la présente invention est un oligomère synthétique formé de 1 à 2 unités d'oses, généralement liées entre elles par liaison glycosidique alpha ou bêta. En d'autres termes, il s'agit d'un disaccharide.

L'oligosaccharide est un disaccharide, plus précisément le sucrose.

Le sel de potassium du sucrose octasulfate est un "oligosaccharide polysulfaté", c'est-à-dire un oligosaccharide dont tous les groupes hydroxyles de chaque ose ont été substitués par un groupe sulfate.

L'oligosaccharide polysulfaté utilisé dans le cadre de la présente demande est le sucrose octasulfate.

L'oligosaccharide polysulfaté utilisé dans le cadre de la présente invention se présente sous forme d'un sel.

Dans le cadre de la présente invention, l'oligosaccharide polysulfaté utilisé est le sel de potassium du sucrose octasulfate.

L'oligosaccharide polysulfaté utilisé dans le cadre de la présente invention peut se présenter sous forme de poudre micronisée ou sous forme solubilisée.

Un exemple d'oligosaccharide polysulfaté utilisé dans le cadre de la présente invention est le sel de potassium du sucrose octasulfate (connu sous l'abréviation KSOS), commercialisé dans le produit Urgotul® Start par les Laboratoires URGO.

### Substance active additionnelle

D'une façon générale, le sel de potassium du sucrose octasulfate selon l'invention pourra être utilisé seul ou en mélange avec un ou plusieurs autres composés oligosaccharides, ou encore en combinaison avec une (ou plusieurs) autre(s) substance(s) active(s).

Les autres composés oligosaccharides pouvant être utilisés en mélange avec le sel de potassium du sucrose octasulfate sont des oligomères synthétiques formés de 1 à 4 unités d'oses, et de préférence de 1 ou 2 unités d'oses, généralement liées entre elles par liaison glycosidique alpha ou bêta. En d'autres termes, il s'agit de mono, di, tri ou tétrasaccharides, et de préférence de mono ou disaccharides.

Il n'y a pas de limitation particulière concernant la nature des unités oses de ces polysaccharides. De préférence, il s'agira de pentoses ou d'hexoses. A titre d'exemple de monosaccharide, on peut citer le glucose, le galactose ou le mannose. A titre d'exemple de disaccharide, on peut citer le maltose, le lactose, le sucrose ou le tréhalose. A titre d'exemple de trisaccharide, on peut citer le mélézitose. A titre d'exemple de tétrasaccharide, on peut citer le stachyose.

De préférence, l'oligosaccharide est un disaccharide, et de préférence encore le sucrose.

Les autres oligosaccharides polysulfaté sont des oligosaccharides dont au moins deux, et de préférence tous les groupes hydroxyles de chaque ose ont été substitués par un groupe sulfate.

De préférence, l'autre oligosaccharide polysulfaté utilisé dans le cadre de la présente demande est le sucrose octasulfate.

Les autres oligosaccharides polysulfatés utilisés dans le cadre de la présente invention peuvent se présenter sous forme de sels ou complexes.

A titre d'exemple de sels, on peut citer les sels de sodium, les sels de calcium, les sels d'argent, ou encore les sels d'acide aminé.

A titre d'exemple de complexes, on peut citer les complexes d'hydroxyaluminium.

Les oligosaccharides polysulfatés utilisés dans le cadre de la présente invention peuvent se présenter sous forme de poudre micronisée ou sous forme solubilisée. De manière générale, les actifs sont choisis parmi les anti-bactériens, les antiseptiques, les anti-douleurs, les anti-inflammatoires, les actifs favorisant la cicatrisation, les agents dépigmentants, les antiprurigineux, les filtres UV, les agents apaisants, les agents hydratants, les agents anti-oxydants, et leurs mélanges.

De manière générale, les actifs sont choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques, des sels d'argent ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de *Centella Asiatica,* la papaïne, les silicones, les huiles essentielles de thym, de niaouli, de romarin et de sauge, l'acide hyaluronique, la metformine" l'Allantoïne, Héma'tîte® (gattefossé), Vitamine C, TEGO® Pep 4-17( evonik), Toniskin® (Silab), Collageneer® (Expanscience), Timecode® (Seppic), Gatuline® skin repair (gattefossé), Panthenol, PhytoCellTec® Alp Rose (Mibelle Biochemistry), Erasyal® (Libragen), Serilesine® (Lipotec), Hétérosides de Talapetraka (Bayer), Stoechiol (Codif), macarose (Sensient), Dermaveil (Ichimaru Pharcos), Phycosaccharide AI (Codif), la metformine ;
- les agents dépigmentants tels que l'acide kojique (Kojic Acid SL^{®} - Quimasso (Sino Lion)), l'Arbutine (Olevatin^{®} - Quimasso (Sino Lion)), le mélange de palmitoylpropyl de sodium et d'extrait de nénuphar blanc (Sepicalm^{®} - Seppic), l'undécylénoyl phénylalanine (Sepiwhite^{®} - Seppic),
- les antiprurigineux : hydrocortisone, enoxolone, diphénhydramine, antihistaminique à *application locale anti H1
- les actifs hydratants tels que Xpermoist® (Lipotec), Acide hyaluronique, Urée, acides gras, Glycérine, Cires, Exossine® (Unipex)
- les filtres UV tels que Parsol® MCX, Parsol® 1789
- les agents apaisants tels que de la camomille, du bisabolol, du Zanthalène®, de l'acide glycyrrhébénique, tanactine (CPN), Calmiskin® (Silab),
- les agents anti-oxydants, tels que la vitamine E.

Selon un mode préféré de réalisation, le sel de potassium du sucrose octasulfate selon l'invention peut être utilisé en combinaison avec un agent anti-oxydant.

### Galénique

Le sel de potassium du sucrose octasulfate utilisé dans le cadre de la présente invention peut être mis en oeuvre au sein d'une formulation galénique, comme par exemple un gel, une solution, une émulsion, une crème, des granules ou des capsules de taille variable allant du nano ou micromètre au millimètre, qui permettra leur application directement au niveau de la plaie. Alternativement, le composé utilisé dans le cadre de la présente invention peut être mis en oeuvre au sein d'une solution pour injection sous-cutanée.

S'il est employé en mélange avec un ou plusieurs autres composés oligosaccharides ou encore en combinaison avec une ou plusieurs autres substances actives, ces composés pourront être incorporés dans la même formulation galénique ou dans des formulations galéniques distinctes.

Bien entendu, la quantité de sel de potassium du sucrose octasulfate selon l'invention utilisée dans la formulation galénique est adaptée en fonction de la cinétique recherchée ainsi que des contraintes spécifiques liées à sa nature, solubilité, résistance à la chaleur, etc.

D'une manière générale, lorsqu'il est utilisé dans une formulation galénique, le sel de potassium du sucrose octasulfate selon l'invention pourra être incorporé en une teneur comprise entre 0,1 et 50% en poids par rapport au poids total de la formulation.

### Pansement

De manière préférentielle, le sel de potassium du sucrose octasulfate utilisé dans le cadre de la présente invention, ou une formulation galénique le contenant, sera intégré à un pansement.

Le sel de potassium de sucrose octasulfate ou une formulation galénique le contenant pourra être incorporé dans un élément quelconque de la structure d'un pansement sous réserve que ce composé puisse entrer directement ou indirectement en contact avec la surface de la plaie.

De préférence et afin de favoriser une action rapide, ce composé (ou une formulation galénique le contenant) sera incorporé dans la couche du pansement qui vient en contact avec la plaie ou déposé sur la surface du pansement qui vient en contact avec la plaie.

Avantageusement, le sel de potassium du sucrose octasulfate (ou une formulation galénique le contenant) pourra ainsi être déposé, de façon continue ou discontinue, sur la surface destinée à venir au contact de la plaie :
- soit sous forme liquide, par exemple par vaporisation d'une solution ou suspension le contenant ;
- soit sous forme solide, par exemple par tamisage d'une poudre le contenant.

La couche ou surface venant en contact avec la plaie pourra être constituée par exemple d'un matériau absorbant telle qu'une mousse absorbante hydrophile en polyuréthane ; un matériau textile telle qu'une compresse, comme par exemple un non tissé, un film, un voile de fibres ; un matériau adhésif absorbant ou non ; une structure interface adhérente ou non.

De façon générale, on pourra jouer sur la galénique ou la structure du pansement pour obtenir un profil de relargage du sel de potassium de sucrose octasulfate spécifique, rapide ou retardé, selon les besoins.

Bien entendu, la quantité de sel de potassium de sucrose octasulfate utilisée dans la formulation galénique ou dans le pansement sera adaptée en fonction de la cinétique recherchée ainsi que des contraintes spécifiques liées à sa nature, solubilité, résistance à la chaleur, etc.

Par pansement, on entend désigner, au sens de la présente demande, tous types de pansements utilisés pour le traitement des plaies.

Typiquement, un pansement comprend au moins une couche ou matrice, adhésive ou non.

Le sel de potassium du sucrose octasulfate selon l'invention, ou une formulation galénique le contenant, peut être incorporé dans un élément quelconque de la structure d'un pansement, par exemple dans la matrice.

De préférence, et afin de favoriser une action rapide, ce composé (ou une formulation galénique le contenant) peut être incorporé dans la couche du pansement qui vient en contact avec la plaie ou déposé sur la surface de la couche du pansement qui vient en contact avec la plaie.

De telles techniques de dépôt sont bien connues de l'homme de l'art et certaines sont par exemple décrites dans la demande de brevet WO 2006/007814.

Dans le cadre de son utilisation dans un élément de pansement, le sel de potassium du sucrose octasulfate selon l'invention sera incorporé en une quantité telle que la quantité de sel de potassium du sucrose octasulfate relarguée dans les exsudats de la plaie soit comprise entre 0,001 g/l et 50 g/l, et de préférence entre 0,01 et 10 g/l.

Selon une variante de l'invention, le sel de potassium du sucrose octasulfate selon l'invention peut être incorporé dans un pansement absorbant à base de fibres gélifiantes, comme par exemple le produit AQUACEL® commercialisé par la société CONVATEC.

Très souvent, lors de la pose de ces pansements, le personnel soignant maintient ces derniers en place à l'aide d'une bande ou recouvre ces derniers d'un élément secondaire tel qu'un second pansement absorbant ou une bande de contention. Il est donc utile que le pansement reste fixé sur la plaie afin que le personnel soignant conserve les mains libres pour positionner ces éléments secondaires. D'une façon générale, tout type d'adhésif couramment employé dans les pansements pourra être utilisé à cet effet.

Afin de ne pas altérer les tissus sains ou les berges de la plaie, notamment lors du retrait du pansement, on préférera un adhésif ayant la propriété d'adhérer à la peau sans adhérer à la plaie.

A titre d'exemple d'un tel adhésif, on peut ainsi citer les adhésifs à base d'élastomères de silicone ou de polyuréthane, tels que les gels de silicone ou de polyuréthane, et les adhésifs hydrocolloïdes.

De tels adhésifs hydrocolloïdes sont notamment constitués d'une matrice élastomérique à base d'un ou plusieurs élastomères choisis parmi les polymères séquencés poly(styrène-oléfine- styrène) en association avec un ou plusieurs composés choisis parmi les plastifiants, tels que les huiles minérales, des résines tackifiantes et, si nécessaire, des antioxydants, dans laquelle est incorporée une quantité, de préférence faible, d'hydrocolloïdes (de 3 à 20% en poids) comme par exemple la carboxyméthylcellulose de sodium ou des polymères superabsorbants comme les produits commercialisés sous la dénomination LUQUASORB® par la société BASF.

Selon un mode préféré de réalisation, le sel de potassium du sucrose octasulfate utilisé dans le cadre de la présente invention, ou une formulation galénique le contenant, sera intégré à un pansement comprenant un adhésif hydrocolloïde, ledit sel de potassium du sucrose octasulfate étant incorporé dans ledit adhésif de préférence en une quantité comprise entre 1 et 15 % en poids, de préférence encore entre 5 et 10 % en poids, par rapport au poids de l'adhésif.

La formulation de tels adhésifs hydrocolloïdes est bien connue de l'homme de l'art et décrite par exemple dans les demandes de brevet FR 2 783 412, FR 2 392 076 et FR 2 495 473.

L'utilisation d'un filet d'adhésif sur le non tissé permet d'une façon particulièrement avantageuse de diminuer ou d'éviter le risque que de petites fibrilles du matériau textile viennent au contact de la plaie et s'accrochent aux tissus, en provoquant ainsi une sensation douloureuse au retrait, voire un obstacle au processus de cicatrisation de la plaie. Elle permet, en outre, de mieux réguler le flux de liquide au niveau du matériau textile et de réduire ou d'éliminer les risques de "gel blocking", résultant de l'utilisation de fibres superabsorbantes, qui limitent de fait la capacité d'absorption du non tissé.

Selon une variante de réalisation préférée de la présente invention, le sel de potassium du sucrose octasulfate selon l'invention est incorporé dans un tel adhésif à une concentration compatible avec sa solubilité et sa résistance à la chaleur.

Sur la base de ces critères, le sel de potassium du sucrose octasulfate selon l'invention est utilisé de préférence en une quantité comprise entre 1 et 15% en poids, et de préférence encore entre 5 et 10% en poids, par rapport au poids total de l'adhésif.

Si l'on souhaite augmenter l'absorption de ce pansement non tissé, on pourra associer ce dernier avec une couche absorbante additionnelle, et de préférence une couche absorbante qui ne gélifie pas, comme en particulier une compresse telle que celle utilisée dans le produit URGOTUL® Duo ou URGOTUL® Trio, une mousse hydrophile absorbante, de préférence une mousse polyuréthane hydrophile présentant une capacité d'absorption supérieure à celle du non tissé telle que celle utilisée dans le produit CELLOSORB®.

Selon un mode préféré de réalisation, le sel de potassium du sucrose octasulfate selon l'invention est incorporé dans un pansement non tissé, associé avec une couche absorbante additionnelle, et de préférence une couche absorbante qui ne gélifie pas, comme en particulier une compresse.

Selon un autre mode préféré de réalisation, le sel de potassium du sucrose octasulfate selon l'invention est incorporé dans un pansement non tissé, associé avec une couche absorbante additionnelle, et de préférence une couche absorbante qui ne gélifie pas, comme en particulier une mousse hydrophile absorbante, de préférence une mousse polyuréthane hydrophile présentant une capacité d'absorption supérieure à celle du non tissé.

Le non tissé et la mousse peuvent être associés par des techniques bien connues de l'homme de l'art, par exemple par calandrage à chaud à l'aide d'une poudre thermofusible à base de polymères TPU/polycaprolactone.

Cette technique est couramment employée pour le liage entre eux de non tissés destinés au marché médical.

Enfin, cette mousse ou le non tissé (lorsque celui-ci est utilisé seul) peuvent être recouverts d'un support pour protéger la plaie de l'extérieur.

Ce support peut être de taille supérieure à celle des autres couches et rendu adhésif de façon continue ou discontinue sur sa face venant en contact avec la plaie afin d'optimiser le maintien du pansement lors de son usage, en particulier si la plaie se situe sur des zones corporelles non planes.

Ce support et son adhésif sont de préférence imperméables aux fluides mais très perméables à la vapeur d'eau afin de permettre une gestion optimale des exsudats absorbés par le pansement et éviter les problèmes de macération.

De tels supports sont bien connus de l'homme du métier et sont constitués par exemple de films respirants et imperméables tels que des films de polyuréthane, des complexes mousse/film ou non tissé/film.

### Additifs

Outre les agents actifs, le sel de potassium du sucrose octasulfate selon l'invention pourra être utilisé en combinaison avec en combinaison avec une (ou plusieurs) autre(s) substance(s) active(s).

Les additifs couramment utilisés dans la préparation des pansements, peuvent notamment être choisis parmi les parfums, les conservateurs, les vitamines, la glycérine, l'acide citrique, etc.

L'activité du sel de potassium du sucrose octasulfate selon l'invention a été mise en évidence dans les exemples non limitatifs suivants.

### EXEMPLES

**Exemple 1** : Mise en évidence de l'effet du sel de potassium du sucrose octasulfate

### (KSOS) sur la différenciation des myofibroblastes

### 1. Culture des Fibroblastes humains dermiques normaux (NHDF)

On a préparé des cultures de fibroblastes humains dermiques normaux (NHDF) en milieu de culture DMEM/F12 (commercialisées par la société Invitrogen) complémenté de 10% de Sérum de Veau Foetal (commercialisé par la société Invitrogen), de 5µg/ml d'insuline (commercialisée par la société Promokine) et de 1 ng/ml de bFGF (commercialisé par la société Promokine).

### 2. Induction de la différenciation

On a ensuite induit la différenciation des fibroblastes en myofibroblastes.

Pour cela, on a mis les cultures de NHDF sur une boite de P100 coatée par du collagène à 5µg/ml dans un milieu DMEM/F12 complémenté par 10% de Sérum de Veau Foetal et additionné de 10ng/mL de TGF-β (commercialisé par la société Promocell), facteur de croissance contrôlant la prolifération et la différenciation des cellules (Témoin différencié sur la figure 1).

On a fait également un témoin non différencié par des cultures en DMEM/F12 complémenté par 10% de Sérum de Veau sans TGB-β.

On a ajouté le KSOS aux cultures témoin et témoin différencié pour son effet sur la différenciation à raison de 2 mg/ml.

On analyse les résultats après 4 jours de culture.

### 3. Mise en évidence de l'inhibition de la différenciation des fibroblastes

Pour mettre en évidence l'inhibition de la différenciation des fibroblastes en myofibroblastes, on a mesuré l'expression de l'ARNm de l'α-SMA par RT-PCR quantitative, et on a détecté la présence de la protéine α-SMA par la méthode de Western Blot. Pour la mesure de l'effet inhibiteur du composé selon l'invention, on a ajouté du KSOS à 2 mg/ml sur les cultures en différenciation.
- Par RT-PCR quantitative : on a procédé à l'extraction des ARNm par la méthode du Trizol, chloroforme et isopropanol.
- Par la méthode de Western Blot : on a préparé des extraits cellulaires totaux et on a réalisé un Western Blot. Les anticorps utilisés sont des anticorps de lapin anti-α-SMA et de souris anti-actine (commercialisés par Promega).

Les résultats de différenciation à J4 démontrent clairement l'induction de l'expression de l'α-SMA dans les cultures en présence de TGF-β et l'inhibition de cette induction lors de l'ajout de KSOS (Figure 1).
- Immunofluorescence : on a préparé des cultures de cellules en plaque 12 puits sur lamelle de verre coatées avec du collagène. On a procédé à la fixation des cellules par du formaldéhyde à 4%, et à la perméabilisation par du Triton. L'immunomarquage est réalisé par une coloration DAPI (conférant une coloration bleue) pour les noyaux cellulaires, un anticorps lapin anti-α-SMA et un anticorps secondaire anti-lapin couplés Cys3 (Commercialisés par Promega).

Le nombre de cellules différenciées sur le nombre total de cellules est déterminé par comptage cellulaire. Le diagramme de la figure 1 (D) donne les pourcentages de cellules différenciées.

Là encore, l'ajout de KSOS provoque une inhibition de la différenciation générée normalement par le TGF-β.

### Exemple 2: Mise en évidence de l'effet du sel de potassium du sucrose octasulfate (KSOS) sur la rétraction des lattices de collagène

### 1 .Préparation de lattices de collagène

Les lattices de collagène ont été obtenues par ensemencement de 0,8.10⁶ fibroblastes de derme humain normaux (NHDF) dans un gel de collagène de type I de concentration 1,3 mg/ml final.

### 2. Mise en évidence de l'effet du sel de potassium du sucrose octasulfate sur la rétraction de lattices de collagène

On a appliqué un pansement (commercialisé sous la dénomination commerciale Urgotul Start) comprenant 7,5% du sel de potassium du sucrose octasulfate à la surface des lattices de collagène.

Une série témoin, sans pansement, a été réalisée pour évaluer la contraction normale du gel de collagène par les NHDF ensemencés.

De même, un témoin positif à été réalisé en ajoutant du TGF-β à une concentration de 10 ng/ml pour visualiser la contraction forte induite par les NHDF fortement différenciés en myofibroblastes.

### On maintient l'incubation jusqu'à 7 jours.

### 3. Résultats

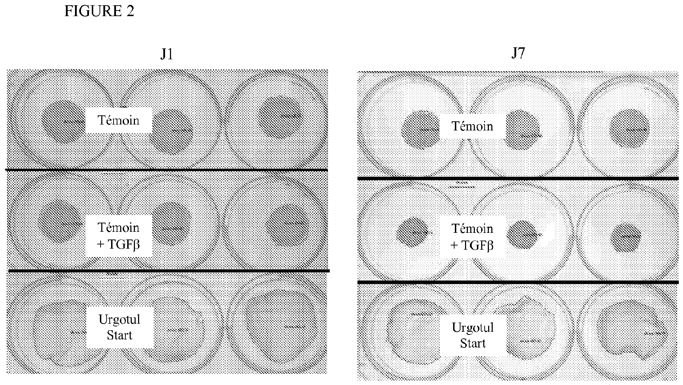

L'observation des effets du sel de potassium du sucrose octasulfate sur la rétraction des lattices de collagène a été effectuée grâce à une analyse photographique (figure 2) et une mesure par analyse d'images de la surface des lattices une fois contractées.

Les résultats des mesures de la surface moyenne des lattices de collagènes apparaissent dans le tableau suivant :

| | Traitement | Moyenne des surfaces (mm²) | % par rapport au témoin |
|---|---|---|---|
| **Jour 1** | Témoin | 229.7 | 100 |
| | Témoin + TGF-β | 217.3 | 95 |
| | Urgotul Start comprenant du KSOS selon l'invention | 533.8 | 232 |
| **Jour 4** | Témoin | 191.0 | 100 |
| | Témoin + TGF-β | 145.9 | 76 |
| | Urgotul Start comprenant du KSOS selon l'invention | 481.4 | 252 |
| **Jour 7** | Témoin | 174.7 | 100 |
| | Témoin + TGF-β | 97 | 56 |
| | Urgotul Start comprenant du KSOS selon l'invention | 484.6 | 277 |

Dans les conditions témoin, les lattices se sont contractées de manière progressive entre le 1^{e} jour et le 7^{e} jour. L'application du pansement Urgotul Start comprenant le sel de potassium du sucrose octasulfate ralentit nettement la contraction des lattices.

Ce résultat démontre que le traitement avec le KSOS limite la contraction des lattices de collagène, ce qui est très positif pour le traitement et la prévention des cicatrices pathologiques avec des niveaux de rétraction des cicatrices importants.

### Exemple 3

On a préparé une formulation de type filmogel à base solvant comprenant un oligosaccharide polysulfaté synthétique selon l'invention ayant la composition suivante :

| **Constituants** | % |
|---|---|
| Nitrocellulose | 12,800 |
| Huile de ricin | 11,000 |
| Ethanol absolu | 24.90 |
| Acétate d'éthyle | 49.70 |
| Filtres UV | 1,500 |
| Sel de potassium du sucrose octasulfate KSOS | 0,100 |

On a dilué la nitrocellulose dans un mélange acétate d'éthyle éthanol absolu. On a ensuite ajouté l'huile de ricin, les filtres UV et le KSOS jusqu'à dissolution pour obtenir une composition de type filmogel.

### Exemple 4

On a préparé une formulation de type filmogel à base aqueuse comprenant un oligosaccharide polysulfaté synthétique selon l'invention ayant la composition suivante :

| **Constituants** | % |
|---|---|
| Eau déminéralisée | 93,200 |
| Epaississant | 0,500 |
| Sorbitol | 2,000 |
| Dextran | 1,000 |
| Sel de potassium du sucrose octasulfate KSOS | 1,000 |
| Méthylparaben | 0,050 |
| Propylparaben | 0,050 |
| Phénoxyéthanol | 0,700 |
| NaOH 10% | 1,500 |

On a dispersé l'épaississant dans l'eau sous vive agitation, puis on a ajouté le sorbitol et le dextran en chauffant à 40°C pour obtenir une meilleure solubilité.

On a ajouté le KSOS, les parabens et le phénoxyéthanol et laissé agiter pour homogénéiser. On a ensuite laissé refroidir jusqu'à température ambiante en arrêtant le chauffage, en ajustant si besoin la perte en eau. Enfin, on a neutralisé avec la soude et laisser agiter 10 minutes avant l'arrêt de l'agitation.

### Exemple 5

On a préparé une formulation sous forme de crème comprenant un oligosaccharide polysulfaté synthétique selon l'invention ayant la composition suivante :

| **Constituants** | % |
|---|---|
| Tensioactif huile-dans-eau | 5,000 |
| Cire émulsionnante | 2,000 |
| Acide stéarique | 1,000 |
| Isononanoate d'isodécyle | 6,000 |
| Huile de silicone (Décaméthyl-Cyclopentasiloxane) | 4,000 |
| Ester émollient (myristyl lactate) | 5,000 |
| Eau déminéralisée | 62,100 |
| Epaississant | 0,300 |
| Glycérine | 5,000 |
| Propylène glycol | 5,000 |
| Sel de potassium du sucrose octasulfate KSOS | 0,500 |
| Conservateur | 1,500 |
| NaOH 10% | 0,600 |
| Surfactant siliconé | 2,000 |

On a dispersé l'épaississant dans l'eau. On a ajouté la glycérine, le propylène glycol, le KSOS et le conservateur et on a homogénéisé. On a chauffé à 70-75°C. Lorsque le mélange atteint 70-75°C, on a ajusté le taux d'eau puis neutralisé avec la soude à 10% et on a ramené la température à 70-75°C.

Dans un même temps, on a mélangé le tensioactif huile-dans-eau, la cire émulsionnante, l'acide stéarique, l'isononanoate d'iodécyle, l'huile de silicone (Décaméthyl-Cyclopentasiloxane), l'ester émollient (myristyl lactate) et on a chauffé à 70-75°C.

Lorsque les 2 mélanges on atteint 70-75°C, on a ajouté le second dans le premier sous vive agitation et on a laissé agiter à chaud pendant 10 minutes.

Puis on a ajouté le surfactant siliconé et on a laissé de nouveau sous agitation à chaud pendant 5 minutes.

Enfin, on a arrêté le chauffage et laissé refroidir à température ambiante, en maintenant une agitation suffisante en fonction de la viscosité du mélange. Le mélange prend un aspect non homogène vers 35°C, mais la crème devient ensuite lisse et brillante.

## Revendications

1. Sel de potassium du sucrose octasulfate pour son utilisation pour traiter des plaies générant des cicatrices pathologiques choisies parmi les cicatrices hypertrophiques, rétractiles ou atrophiques.

2. Sel de potassium du sucrose octasulfate pour son utilisation pour inhiber la différentiation des fibroblastes en myofibroblastes au cours de la cicatrisation de plaies générant des cicatrices pathologiques choisies parmi les cicatrices hypertrophiques, rétractiles ou atrophiques.

3. Sel de potassium du sucrose octasulfate pour son utilisation selon la revendication 1 ou 2, **caractérisé en ce qu'**il est associé à une ou plusieurs autres substances actives choisies parmi les anti-bactériens, les antiseptiques, les anti-douleurs, les anti-inflammatoires, les actifs favorisant la cicatrisation, les agents dépigmentants, les antiprurigineux, les filtres UV, les agents apaisants, les agents hydratants et les agents anti-oxydants et leurs mélanges.

4. Sel de potassium du sucrose octasulfate pour son utilisation selon la revendication 3, **caractérisé en ce que** l'autre substance active est choisie parmi les agents anti-oxydants.

5. Sel de potassium du sucrose octasulfate pour son utilisation selon l'une quelconque des revendications précédentes, **caractérisé en ce qu'**il est présent dans une formulation galénique, comme par exemple un gel, une solution, une émulsion, une crème, des granules ou des capsules permettant une application directement au niveau de la plaie, de préférence en une quantité comprise entre 0,1 et 50 % en poids, par rapport au poids total de la formulation.

6. Sel de potassium du sucrose octasulfate pour son utilisation selon l'une quelconque des revendications précédentes, **caractérisé en ce que** ledit sel de potassium du sucrose octasulfate, ou une formulation galénique le contenant, est intégré à un élément d'un pansement, de préférence en une quantité telle que la quantité du sel de potassium du sucrose octasulfate relarguée dans les exsudats de la plaie soit comprise entre 0,001 g/l et 50 g/l, et de préférence encore entre 0,01 et 10 g/l.

7. Sel de potassium du sucrose octasulfate pour son utilisation selon la revendication 6, **caractérisé en ce que** ledit pansement est un pansement non tissé, associé avec une couche absorbante additionnelle, et de préférence une couche absorbante qui ne gélifie pas, comme en particulier une compresse.

8. Sel de potassium du sucrose octasulfate pour son utilisation selon la revendication 6, **caractérisé en ce que** ledit pansement est un pansement comprenant un non tissé, associé avec une couche absorbante additionnelle, et de préférence une couche absorbante qui ne gélifie pas, comme en particulier une mousse hydrophile absorbante, de préférence une mousse polyuréthane hydrophile présentant une capacité d'absorption supérieure à celle du non tissé.

9. Sel de potassium du sucrose octasulfate pour son utilisation selon l'une quelconque des revendications 6 ou 7, **caractérisé en ce que** ledit pansement comprend un adhésif hydrocolloïde et **en ce que** ledit sel de potassium du sucrose octasulfate est incorporé dans ledit adhésif, de préférence en une quantité comprise entre 1 et 15 % en poids, de préférence encore entre 5 et 10 % en poids, par rapport au poids de l'adhésif.

10. Sel de potassium du sucrose octasulfate pour son utilisation selon l'une quelconque des revendications précédentes, pour son utilisation pour traiter des plaies chez un sujet présentant une prédisposition au développement de cicatrices hypertrophiques, rétractiles ou atrophiques.

11. Sel de potassium de sucrose octasulfate pour son utilisation pour traiter des plaies générant des cicatrices pathologiques choisies parmi les cicatrices d'acné sévère.

## Patentansprüche

1. Kaliumsalz von Sucroseoctasulfat zur Verwendung zur Behandlung von Wunden, die pathologische Narben erzeugen, ausgewählt aus den hypertrophen, retraktilen oder atrophen Narben.

2. Kaliumsalz von Sucroseoctasulfat zur Verwendung zur Differenzierung von Fibroblasten in Myofibroblasten während der Wundheilung von Wunden, welche pathologische Narben erzeugt, ausgewählt aus hypertrophen, retraktilen oder atrophen Narben.

3. Kaliumsalz von Sucroseoctasulfat zur Verwendung nach Anspruch 1 oder 2, **dadurch gekennzeichnet, dass** es assoziiert ist mit einem oder mehreren anderen Wirkstoffen, ausgewählt aus antibakteriellen Mitteln, Antiseptika, Schmerzmitteln, entzündungshemmenden Mitteln, Mitteln, die die Wundheilung begünstigen, Depigmentierungsmitteln, antipruritischen Mitteln, UV-Filtern, reizlindernden Mitteln, hydratisierenden Mitteln und Antioxidantien und ihren Gemischen.

4. Kaliumsalz von Sucroseoctasulfat zur Verwendung nach Anspruch 3, **dadurch gekennzeichnet, dass** der andere Wirkstoff ausgewählt ist aus Antioxidantien.

5. Kaliumsalz von Sucroseoctasulfat zur Verwendung nach einem der vorhergehenden Ansprüche, **dadurch gekennzeichnet, dass** es in einer galenischen Formulierung vorliegt, wie zum Beispiel in einem Gel, einer Lösung, einer Emulsion, einer Creme, Granalien oder Kapseln, welche eine direkte Anwendung auf der Wundstelle erlaubt, vorzugsweise in einer Menge, umfassend zwischen 0,1 und 50 Gew.-%, bezüglich des Gewichts der Gesamtformulierung.

6. Kaliumsalz von Sucroseoctasulfat zur Verwendung nach einem der vorhergehenden Ansprüche, **dadurch gekennzeichnet, dass** die Kaliumsalz von Sucroseoctasulfat oder eine galenische Formulierung, die sie enthält, integriert ist in ein Wundverbandmittel, vorzugsweise in einer Menge, dass die Menge dieser Kaliumsalz von Sucroseoctasulfat, die abgegeben wird in die Wundsekrete zwischen 0,001 g/l und 50 g/l, und noch bevorzugter zwischen 0,01 und 10 g/l umfasst.

7. Kaliumsalz von Sucroseoctasulfat zur Verwendung nach Anspruch 6, **dadurch gekennzeichnet, dass** der Verband ein Verband aus nichtgewebtem Material ist, verbunden mit einer zusätzlichen Absorptionsschicht, und vorzugsweise mit einer Absorptionsschicht, die nicht geliert, wie insbesondere eine Kompresse.

8. Kaliumsalz von Sucroseoctasulfat zur Verwendung nach Anspruch 6, **dadurch gekennzeichnet, dass** der Verband ein Verband ist, umfassend ein nichtgewebtes Material, verbunden mit einer zusätzlichen Absorptionsschicht, und vorzugsweise mit einer Absorptionsschicht, die nicht geliert, wie insbesondere ein hydrophiler absorbierender Schaum, vorzugsweise ein hydrophiler Polyurethanschaum mit einer Absorptionskapazität, die über der von einem nichtgewebten Material liegt.

9. Kaliumsalz von Sucroseoctasulfat zur Verwendung nach einem der Ansprüche 6 oder 7, **dadurch gekennzeichnet, dass** der Verband eine hydrokolloidale Klebemasse umfasst, und dadurch, dass das Kaliumsalz von Sucroseoktosulfat in der Klebemasse enthalten ist, vorzugsweise in einer Menge, umfassend zwischen 1 und 15 Gew.-%, noch bevorzugter zwischen 5 und 10 Gew.-%, bezüglich des Gewichts der Klebemasse.

10. Kaliumsalz von Sucroseoctasulfat zur Verwendung nach einem der vorhergehenden Ansprüche, zur Verwendung zur Behandlung von Wunden eines Patienten, der eine Prädisposition zur Entwicklung von hypertrophen, retraktilen oder atrophen Narben aufweist.

11. Kaliumsalz von Sucroseoctasulfat zur Verwendung zur Behandlung von Wunden, die pathologische Narben entwickeln, ausgewählt aus Narben schwerer Akne.

## Claims

1. Potassium salt of sucrose octasulfate for use in treating wounds resulting in pathological scars chosen from hypertrophic, retractile or atrophic scars.

2. Potassium salt of sucrose octasulfate for use in inhibiting the differentiation of fibroblasts into myofibroblasts during the healing of wounds resulting in pathological scars chosen from hypertrophic, retractile or atrophic scars.

3. Potassium salt of sucrose octasulfate for use according to claim 1 or 2, **characterized in that** it is combined with one or more other active substances chosen from antibacterials, antiseptics, painkillers, anti-inflammatories, active agents which promote healing, depigmenting agents, antipruritics, UV-screening agents, calmatives, moisturizing agents and antioxidants, and mixtures thereof.

4. Potassium salt of sucrose octasulfate for use according to claim 3, **characterized in that** the other active substance is chosen from antioxidants.

5. Potassium salt of sucrose octasulfate for use according to any one of the preceding claims, **characterized in that** it is present in a galenic formulation, for instance a gel, a solution, an emulsion, a cream, granules or capsules allowing application directly to the wound, preferably in an amount of between 0.1% and 50% by weight relative to the total weight of the formulation.

6. Potassium salt of sucrose octasulfate for use according to any one of the preceding claims, **characterized in that** said potassium salt of sucrose octasulfate, or a galenic formulation containing it, is integrated into a component of a dressing, preferably in an amount such that the amount of this potassium salt of sucrose octasulfate released into the wound exudates is between 0.001 g/l and 50 g/l, and more preferably between 0.01 and 10 g/l.

7. Potassium salt of sucrose octasulfate for use according to claim 6, **characterized in that** said dressing is a nonwoven dressing, combined with an additional absorbent layer, and preferably an absorbent layer which does not gel, such as, in particular, a compress.

8. Potassium salt of sucrose octasulfate for use according to claim 6, **characterized in that** said dressing is a dressing comprising a nonwoven, combined with an additional absorbent layer, and preferably an absorbent layer which does not gel, such as, in particular, an absorbent hydrophilic foam, preferably a hydrophilic polyurethane foam with an absorption capacity greater than that of the nonwoven.

9. Potassium salt of sucrose octasulfate for use according to claim 6 or 7, **characterized in that** said dressing comprises a hydrocolloid adhesive and **in that** said potassium salt of sucrose octasulfate is incorporated into said adhesive, preferably in an amount of between 1% and 15% by weight, more preferably between 5% and 10% by weight, relative to the weight of the adhesive.

10. Potassium salt of sucrose octasulfate for use according to any one of the preceding claims, for use in treating wounds in a subject who has a predisposition to developing hypertrophic, retractile or atrophic scars.

11. Potassium salt of sucrose octasulfate for use in treating wounds resulting in pathological scars chosen from severe-acne scars.
